# EUROPEAN PATENT SPECIFICATION

(11) **EP 3 542 740 B1**
(45) Date of publication and mention of the grant of the patent: **30.04.2025**
(21) Application number: 18162744.9
(22) Date of filing: 20.03.2018
(51) Int. Cl.: A61B 17/32, A46B 15/00, A61N 7/00, A61B 90/98

(54) **DEVICE FOR ULTRASOUND WOUND CLEANING**
VORRICHTUNG ZUR ULTRASCHALLWUNDREINIGUNG
DISPOSITIF DE NETTOYAGE DE PLAIES PAR ULTRASONS

(43) Date of publication of application: 25.09.2019
(73) Proprietor: BSN medical GmbH, 22761 Hamburg (DE)
(72) Inventor: Kaufmann, Steffen, 21357 Barum (DE); Rettmer, Sven, 21745 Hemmoor (DE); Lafendt, Sören, 22765 Hamburg (DE); Schütz, Patrick, 12159 Berlin (DE)
(74) Representative: Cohausz & Florack

(56) References cited:
- US-A1- 2014 276 469
- US-A1- 2017 072 224

## Description

### Field of the invention

The invention relates to a medical device for wound cleaning in form of a hand-held device and a cleaning attachment reversibly attachable to the hand-held device and drivable by an electric motor and/or a sonotrode, whereby the hand-held device comprises an ultrasound generating unit consisting of a frequency generator and a sonotrode. The invention further relates to a wound cleaning system consisting of the wound cleaning device of the invention and a transmission medium in form of a gel or an aqueous fluid for transmitting the ultrasound waves onto the tissue to be treated.

### Background of the invention

Chronic wounds present a significant individual and societal burden in their cost of care and they reduce patient quality of life. Key components of wound care include measures as debridement, irrigation and wound cleaning. Appropriate care removes necrotic tissue and reduces wound bioburden to enhance wound healing. Physical cleaning with debridement and irrigation is of documented efficacy.

Debridement is the surgical excision of dead, devitalized, and contaminated tissue and/or the removal of foreign matter from a wound. Debridement is advantageous because it cleans the wound and lowers the bacterial count of wound tissues, thereby facilitating the healing process.

Some wound cleaning and debridement devices with relatively large dimensions harbor the risk of removing unintended tissue from the subject or damaging the unintended tissue. Furthermore, they require a massive and costly base unit which is difficult to transport. Hence, there is a need for tissue removal devices which have small dimensions and improved functionality over existing products and procedures which allow them to more efficiently remove wound plaque as well as tissue from the patient.

Hand-held wound debridement devices often include an irrigation system to provide pressurized streams of fluid to dislodge unwanted tissue and foreign matter from a wound and a suction system to collect the dislodged matter and irrigation fluid. These hand-held wound debridement systems generally provide an efficient and effective means of debriding a wound. However, the use of such systems can, disadvantageously, result in the splashing of irrigation fluid and body fluids from the patient onto, e. g. the healthcare professional operating the debridement device.

Ultrasound-supported wound cleaning devices are known in the prior art. These devices commonly produce alternating low-pressure and high-pressure waves in a water jet or a spray mist leading to the formation and violent collapse of small vacuum bubbles. This phenomenon is termed cavitation and causes high speed impinging liquid jets and strong hydrodynamic shear-forces. The liquid represents a constant medium for ultrasound transmission and supports the removal of the loosened material, but hereby also increases aerosol formation and generation of potentially infectious fluid.

The mechanically operating wound cleaning devices in their functionality represent a tightrope walk: on one side, they must be efficient enough to remove also tenacious debris, on the other hand they should not be too strong so that non-necrotic wound tissue is damaged, and pain and discomfort is caused to the patients.

Aerosol formation is a severe problem since it requires a subsequent professional decontamination of the treatment area which might include the whole room. Hence, this precludes a use of said devices in the homecare-setting or at the patient's bedside. In most cases, these complications prevent a treatment of patients with highly infectious germs, such as multiresistant staphylococcus aureus (MRSA), since the risk of a dissemination of germs is too high. In order to reduce the aerosol derived contamination, certain companies offer shield solutions such as the UAW ClearShield from Söring GmbH (Quickborn, Germany). These shields have the disadvantage that they impair the visibility and the handling of the user and furthermore increase the treatment costs.

The patent DE 10 2015 201 092 B4 relates to a system for ultrasound treatment of open wounds and teaches a manual device with a replaceable and disposable cleaning attachment with an integrated sonotrode. It is the groundwork for the toothbrush like debridement device Curason distributed by Curasonix. The sonotrode transmits the generated ultrasound waves via a pad being centrally located within a brush. The reduced performance of the disposable sonotrode together with the dampening of the ultrasound waves via the rubber pad leads a reduced energy density, so it is not possible to generate a sufficient amount of cavitation bubbles. Furthermore, although the device works without a steady liquid supply, the generation of aerosol is only partially impaired since the ultrasound-pad is freely accessible and the oscillating brushes tend to whirl up further aqueous liquid. US 2017/072224 A1 and US 2014/276469 A1 also disclose wound cleaning devices of the prior art.

There is still a need for an improved wound cleaning/debridement device that addresses the different complex requirements for an effective and safe wound cleaning. The objective of the present invention thus is to provide a wound cleaning device which overcomes at least one of the above-mentioned disadvantages.

This problem is solved by provision of a wound cleaning device according to claim 1.

### Summary of the invention:

Claim 1 defines the invention and dependent claims disclose embodiments. No surgical methods are claimed per se. In a first aspect the invention, as defined by claim 1, provides a medical device for wound cleaning in the form of a hand-held device comprising:
(a) an ultrasound generating unit comprising a frequency generator and a sonotrode;
(b) optionally an electric motor for driving a cleaning attachment;
(c) a power source for operating the electric motor and/or the sonotrode;

and a cleaning attachment which is reversibly attached to the hand-held device and drivable by the electric motor and/or the sonotrode and comprises an abrasive unit and is devoid of a sonotrode;
whereby the sonotrode is arranged to transfer the ultrasound energy onto the tissue to be treated.

The wound cleaning device of the present invention has several advantages over wound cleaning devices known in the prior art.

The implementation of a simultaneous mechanical and electrical ultrasound-debridement-technology in a hand-held device allows a significant saving of time for the cleaning and treatment of chronic wounds and leads to an extension of the treatable patient cohorts.

Due to the efficient ultrasound-generation, the cleaning efficacy is high enough to clean also necrotic wounds thoroughly and reliably, albeit gently.

By enclosing the sonotrode through the disposable cleaning attachment, the problem of the aerosol formation is properly addressed. This barrier prevents the formation of hazardous aerosols when contacting the wound.

In addition, the sonotrode can be controlled in a way that it is shut off as soon as the contact to the patient is missing (e.g. via a pressor sensor or an analysis of the emitted energy) so that the emission of germs is minimized.

In order to avoid the constant use of an irrigation fluid, but providing sufficient liquid for generation of cavitation bubbles, the disposable cleaning attachment preferably comprises a liquid reservoir in the form of a grid, foam, sponge or textile fabric. This liquid reservoir is positioned in front of the sonotrode thereby bridging the space between sonotrode and treatment site.

Since the device combines two active principles, namely the ultrasound and the oscillatory/rotary movement of the cleaning attachment, which can be regulated and controlled independently from each other, the device can easily be adapted to the specific requirements of the underlying wound cleaning situation. Hence, it offers an enormous flexibility of application while addressing the divergent requirements of wound cleaning and debridement.

Since the cleaning attachment is devoid of a sonotrode, it is easy to manufacture, reliably in use and cost-efficient to produce.

The cleaning device of the invention can be easily equipped with additional components for enhancing safety or efficiency.

Since the wound cleaning device of the invention is based on known components, it can be easily produced in a cost-efficient manner.

### Detailed description of the invention and other disclosures

In a preferred embodiment of the invention the hand-held device has a water-tight housing in which the ultrasound-generating unit, the optional electric motor and the power source are integrated.

According to the invention, the cleaning attachment can be driven by the electric motor and/or the sonotrode and thus provides three different embodiments for drive systems:
Embodiment 1: The cleaning attachment is driven by the electric motor. In this embodiment, the electric motor leads to a rotatory, oscillatory or rotational-oscillatory movement of the attachment. The primary role of the sonotrode is hereby the generation of cavitation bubbles supporting the mechanical debridement mechanism.
Embodiment 2: The cleaning attachment is driven by the sonotrode. In this_embodiment, the ultrasound as generated by the sonotrode induce an oscillatory movement of the cleaning attachment. In this embodiment the sonotrode still has the further function of transmitting ultrasound energy to the treatments site, such as by generating cavitation bubbles.
Embodiment 3: In a third embodiment, the cleaning attachment is driven by both, electric motor and the sonotrode, so that the (preferable rotational) movement as generated by the electric motor is combined with and superimposed by an oscillatory movement as generated by the sonotrode.

The releasable cleaning attachment can be driven by an electro motor resulting in an oscillatory movement with an angle of between 10° and 360° perpendicular to the treatment plane.

Alternatively, the releasable cleaning attachment can be driven by an electro motor resulting in a rotational-oscillatory movement with an angle of more than 360° perpendicular to the treatment plane.

Preferred frequencies for the oscillatory movement of the cleaning attachment are less than 100 Hz, preferably between 1 and 10 Hz and more preferably at 5 Hz.

The water-tight housing is suitably made from metal or plastic. Suitable polymer for the housing are polyacrylate, polypropylene, rubber or silicone.

The water tight housing is preferably a housing that is water and dust resistant with an IP68 rating for water resistance up to 1.5 m deep for 30 minutes in order to be used in the shower or in the bathtub.

In another embodiment of the invention, the housing is made from materials with built-in antimicrobial and antibacterial properties that help to resist the growth of bacteria, mold, mildew, fungi, viruses and other microbes.

In one embodiment of the invention the housing of the medical device comprises a grip portion having a contoured profile to enhance grip and facilitate manual control of the device by a user.

The ultrasound-generating unit is connected to a power supply unit that generates alternating current oscillating at ultrasonic frequency. This alternating current is applied onto the sonotrode to generate ultrasonic vibrations and applies this vibrational energy using the transmission medium onto the wound site.

The ultrasound-generating unit is preferably arranged for the generation of an alternating current with frequencies in the range of between 16 kHz and 1 GHz, preferable between 18 kHz and 1 MHz and more preferable between 20 kHz to 100 kHz.

The preferred signal type of the ultrasound-generating unit is a square wave signal, whereby other signal types could also be used such a sinusoidal signals or chirp signals.

The alternating current is optionally applied in a pulsed fashion or continuous fashion as desired.

In a further embodiment of the invention, the electric motor of the medical device is arranged by use of additional drive components to generate an oscillating, oscillatory-rotating or rotating motion of the cleaning attachment.

The motion of the cleaning attachment is preferably configurable with regard to frequency or speed. This enables a specific adoption to the therapeutic requirement.

In a further embodiment, the electric motor of the medical device, by using additional drive components, is arranged to generate a combined vertical and horizontal motion of the cleaning attachment. The additional horizontal motion can be used to detach debris which is difficult to remove.

In a further embodiment the electric motor together with additional drive components is arranged to generate opposing motions, so that one part of the cleaning attachment is moved clockwise, and another part is moved anticlockwise. When the cleaning attachment has the preferred annular form, it could therefore comprise an outer ring and an inner ring which are movable in opposing directions.

The additional drive components are used to provide speed and torque conversions from the rotating electric motor to the cleaning attachment. The skilled person can select the appropriate drive components from a huge repertoire of technical solutions. Exemplary drive components include an eccentric, a cam lever or a gear.

The drive components preferably include bearings for smooth and effortless transmission of the motion. Especially preferred are needle roller bearings which are roller bearings with cylindrical rollers, which are thin and long in relation to their diameter (needles).

The abrasive unit of the cleaning device forms a cleaning surface and is preferably designed as textile pad, foam, sponge, brush head, lamellar structure, rubber pad or a combination thereof. The cleaning surface and the listed different designs are preferably flexible in order to adapt their surface to the wound surface.

In a preferred embodiment, the cleaning surface is the surface of a textile pad or brush or any combination of these two.

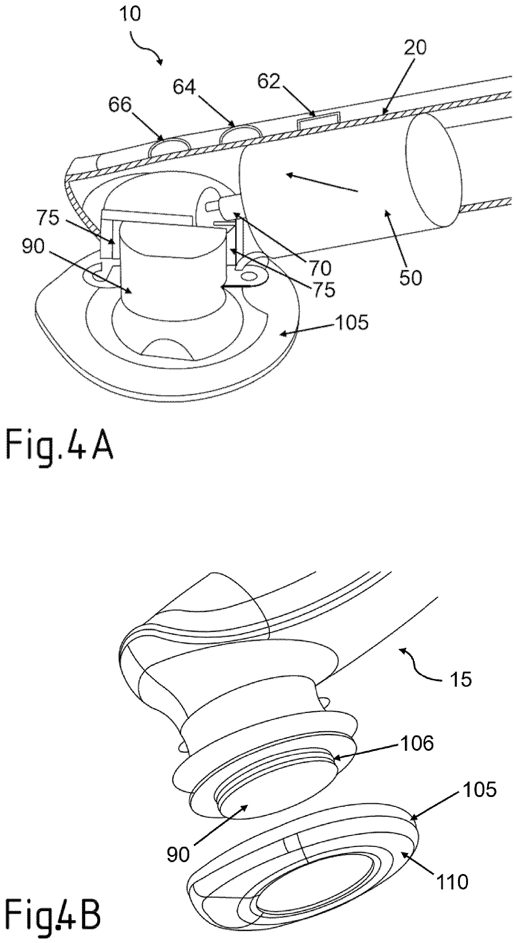

In a further embodiment, the cleaning surface of the attachment has an irregular (i.e. a non-circular) outer contour with a narrow and a broad part. This allows the cleaning of large wounds with the broad part of the surface as well as the cleaning of smaller wounds with the narrow part of the surface. In a preferred embodiment, this is achieved by a drop-shaped outer contour (see Fig. 4) whereby the circle-derived basic structure can be used for larger wounds and the tip of the drop can be used for precise cleaning of smaller wounds. In addition, this contour also allows an easy recognition of the necessary orientation when connecting the cleaning attachment to the hand-held device. Additionally, the pointed tip allows a more precise cleaning of smaller wounds or wound areas with enhanced cleaning or debridement requirements.

In a preferred embodiment the non-circular outer contour of the cleaning attachment is combined with the overall annular structure, so the ring-shaped attachment has an irregularly formed ring. In a more preferred embodiment the irregular shape is only given at the outer contour, so that the central circular opening fits to the circular ultrasound-transmitting surface of the sonotrode and the outer protrusion of the ring supports the above described precise cleaning.

In another embodiment, the cleaning attachment has a mark or label that indicates the correct orientation for attachment to the hand-held device. This mark or label can be a color mark or an engraving, but it can be also provided by the above disclosed irregular shape of the cleaning attachment surface such as the tip of the drop-shaped contour.

According to the invention the cleaning attachment is reversibly attachable, meaning that it can be easily attached and detached without excessive force keeping the hand-held device intact for further use.

In one embodiment the cleaning surface is a multilayered laminate structure. Preferred is a foam with a textile layer as top wound-contacting layer.

The textile layer is preferably made of microfibers enabling a better removal of debris and bacteria.

The laminate structure and preferably its textile layer can be equipped with antimicrobial agents or can release antimicrobial agents.

In one embodiment of the invention the abrasive unit has a soft cleaning surface. A soft surface as provided by a foam or a sponge has a flexible nature and makes it capable of conforming to irregularly shaped wounds. This capability results in more efficient debridement and at the same time, reduces the spray of transmission medium and/or body fluids from the area being debrided. Furthermore, the absorbent nature of the foam or sponge allows it to absorb excess fluid at the debridement site, thereby resulting in a more efficient debridement procedure.

According to the invention, the cleaning attachment is reversibly attachable to the hand-held device. For this attachment, every type of connection enabling a reversible connection can be used and allowing a firm connection even for rotating and/or oscillating conditions. Non-excluding examples comprised of a snap-fit, a gear rim, a magnetic lock or a bayonet connection. The snap fit is the preferred coupling.

In a preferred embodiment of the invention, the surface of the sonotrode defining the radiation direction is coplanar to the cleaning surface of the cleaning attachment. By virtue of this design, the energy transfer from the sonotrode to the skin as treatment site is maximized.

In one embodiment of the invention the sonotrode works according the magneto-restrictive or piezo-ceramic principle.

The sonotrode is preferably arranged for the generation of ultrasound frequencies in the range of between 16 kHz and 1 GHz, preferable between 18 kHz and 1 MHz and more preferable between 20 kHz to 100 kHz.

The sonotrode is optionally adapted to generate longitudinal vibration, transverse vibration, or combination thereof at desired frequencies.

The sonotrode is preferably arranged for the generation of ultrasound frequencies with an intensity from 0.25 W/cm² to 3 W/cm².

In a preferred embodiment of the invention, the cleaning attachment comprises an central opening giving access to the sonotrode. This design enables the direct, unimpeded transfer of the ultrasonic waves from the sonotrode to the wound site.

In a more preferred embodiment the cleaning attachment is preferably of annular design. This means, that the cleaning attachment has a ring-shaped or toroidal form with a central hole, preferably a clearance hole for direct transmission of the ultrasonic energy generated from the sonotrode to the wound site.

In a specific embodiment, the opening is at least partially filled with a fluid-absorbing structure or covered by a fluid-absorbing structure. This fluid-absorbing structure can be presoaked with the transmission medium before treatment and allows an optimal transmission of ultrasonic energy and generation of cavitation bubbles.

Preferably, the fluid-absorbing structure is a grid, foam, sponge, or a textile fabric. Hereby a textile fabric or foam is preferred. Both are rather inexpensive materials and can absorb sufficient amount of liquids.

In one embodiment of the invention, the hand-held device or the cleaning attachment comprises one or more of the following components:
(a) a means for identification of the cleaning attachment;
(b) an On/Off switch;
(c) a device for controlling the operation mode;
(d) a device for measuring the application duration;
(e) a sensor for measuring the contact force;
(f) a sensor for measuring the mechanical work;
(g) a sensor for measuring the bioimpedance;
(h) a temperature sensor;
(i) a sensor for measuring the released ultrasound energy;
(j) an acoustic signaler;
(k) a visual signaler;
(l) a UV-light source;
(m) a display unit for displaying operational parameters;
(n) unit for wireless communication;
(o) a liquid container with integrated rinsing;
(p) a housing having rounded corners for reducing microbiological contamination;
(r) port for wired connection.

Preferably, the medical device comprises means for identification of the cleaning attachment.

The device suitably has an On/Off switch for switching an and switching off the device. The device is preferably equipped with an auto switch-off functionality that will be activated when the device is not operating for a selected period of time.

The medical device can further comprise a device for controlling the operation mode in order to prevent any unintended or unsafe use.

The medical device can further comprise a device for measuring the application duration; so that the end of the programmed or preselected operation time is indicated to the user and/or could be used to switch off the device.

The medical device can further comprise a sensor for measuring the contact force. Since the contact force is an important parameter for an efficacious and safe wound cleaning, the control of this parameter is of importance.

The medical device can further comprise a sensor for measuring the mechanical work so that the achievement of a programmed or preselected mechanical work limit is indicated to the user and/or could be used to switch off the device.

In a preferred embodiment, the medical device comprises a sensor for measuring the bioimpedance. By determination of the bioimpedance several physiologically relevant aspects can be assessed such as wound condition, the amount of transmission medium or the contact quality between the device and the wound.

In a further preferred embodiment, the medical device comprises a temperature sensor measuring the temperature at the sensor and/or at the skin surface so that a temperature exceeding a critical temperature is indicated to the user and/or could be used to switch off the device.

The medical device can further comprise a sensor for measuring the released ultrasound energy so that the achievement of a programmed or preselected ultrasound energy limit is indicated to the user and/or could be used to switch off the device.

The medical device is suitably equipped with an acoustic and/or visual signaler to indicate relevant operating parameters and/or warning signals to the user.

In a further embodiment of the invention the medical device comprises a UV-light source, being preferably an LED. The UV light that is directed on the treatment site and/or on the cleaning attachment could kill microorganisms. For this germicidal purpose the UV light is short-wave UV-C having a wave length of between 100 and 280 nm, and preferably of 254 nm.

The medical device is suitably equipped with display unit for displaying operational parameters.

In a preferred embodiment the medical device of the invention further comprises a unit for wireless communication. By use of the wireless communication unit, the medical device of the invention can communicate with external devices such as the charging unit and can inter alia perform software updates, configuration adjustments, readout of log files.

In another embodiment the medical device of the invention comprises a liquid container for integrated rinsing. In comparison to other irrigation techniques this still allows for a portable device. Notably, a potential increase in the risk of aerosol formation can be prevented by a specific form of the cleaning attachment, e.g. by encircling the sonotrode by the cleaning attachment.

The container is preferably a separate container which can be reversibly attached to the hand-held device.

In a preferred embodiment the medical device further comprises a pumping system and a tubing system for transporting the irrigation liquid to one or more openings within the hand-held device or the cleaning attachment in order to provide a proper irrigation of the wound.

The medical device can further comprise a container for collecting irrigation fluid and/or debris which is collected by the medical device, preferably by use of a suction device integrated within the hand-held device. This collection container can be a separate container, or it can be identical to the container for integrated rinsing.

Suitably, the medical device comprises a housing having rounded corners for reducing microbiological contamination, as well as easy and fast cleaning of the device.

In a preferred embodiment the medical device has a port for wired connection.

In a preferred embodiment of the invention, the energy source of the medical device is an accumulator, which is preferably wireless loadable by an inductive charging unit.

In a further embodiment of the invention, the cleaning attachment comprises a hinge, which is positioned such during use the attachment can pivot freely with regard to the hand-held device around a pivot axis (Ap) that extend substantially perpendicular to the oscillation and/or rotation axis (Ao_{R}) of the cleaning attachment.

In the second aspect the invention provides a wound cleaning system consisting of a medical device for wound cleaning according to the invention and a transmission medium in form of a (hydro)gel or an aqueous fluid for transmitting the ultrasound waves onto the tissue to be treated.

The transmission medium functioning as a coupling medium is necessary since the sonotrode has no direct contact to the tissue and so this medium transmits the ultrasound waves from the sonotrode to the skin surface and furthermore is required as a medium of the generation of cavitation bubbles. Notably, ambient air is a relatively poor medium for the propagation of ultrasonic waves.

The transmission medium can also provide a cooling effect which dissipates some of the heat energy produced by the sonotrode.

Examples for suitable transmission media are water, phosphate buffer, ringer solution, isotonic saline solution, wound cleaning liquids such as Prontosan^{®} (Fa. B. Braun Melsungen AG, Melsungen, Germany) and a variety of gels, such as gels used in wound bed preparation like the Cutimed Gel.

The skilled person will know how and in which amount the transmission medium has to be applied in order to minimize any energy loss.

By use of either the liquid reservoir filled with the transmission medium or a pre moistened cleaning attachment and/or wound site, the medical device of the invention is efficacious as classical contact ultrasound treatment and thereby leads to the beneficial effects that have been reported from contact ultrasound treatment such as: local improvement of the blood circulation, heating of the tissue, accelerated enzyme activity, muscle relaxation, pain reduction, and enhancement of natural healing processes. Furthermore, the chances of user errors are reduced greatly so that the risk of injury is minimized.

The transmission medium is preferably applied before the treatment onto the wound to be cleaned or the liquid reservoir or the hole in the annular cleaning attachment.

When using the medical device of the invention, the treatment is applied to a skin surface for a time period of between 1 and 30 minutes. The treatment can be performed once daily, or at longer intervals such as every other day, or two or three times the week.

In a preferred embodiment, the cleaning attachment can release one or more active agents which support the wound cleaning.

Examples for active agents are:
- Antibiotics in order to kill wound-derived microorganisms such as sulphonamides;
- Analgesics for pain control elicited by the cleaning procedure such as NSAIDS like diclofenac, ibuprofen or ketoprofen;
- Local anaesthetics for numbing the tissue to be treated, such as lidocaine, mepivacaine, prilocaine, procaine, syntocaine, tetracaine, gingicaine, articaine, bupivacaine, butanilicaine, chloroprocaine, or for example, polidocanol;
- Anti-inflammatory substances that, as the case may be, exhibit secondary analgesic properties, such as hormones, particularly cortisone and corticoids, specifically glucocorticoids (e.g. cortisone, cloprednol, prednisone, prednisolone, methylprednisolone, deflazacort, fluocortolone, triamcinolone, dexamethasone, betamethasone) and mineralocorticoids (e.g. aldosterone, desoxycorticosterone, fludrocortisone);
- Surfactants for lowering the surface tension of the transmission medium or the wound exudate;
- A water-in-oil-emulsion with wool wax alcohols as oil phase. Hereby the wool wax alcohols are preferably a mixture of aliphatic C18-C20 alcohols and sterols with a cholesterol content of at least 30 % and are also known under "Eucerit";
- Oil, hydrogel or creme for promoting the wound healing such as Niendorfs oil;
- Oil, hydrogel or creme for skin care.

For medical use, it is required that the cleaning attachment is provided (most preferably in a moistened ready-to-use form) in sterile form. This can be achieved by packaging the sterile product in a bacterial tight material with a marking on the packing that the product is sterilized. Bacterial tight materials are well known to the person skilled in art. Since the cleaning attachment is preferably a disposable product, a post-treatment cleaning and disinfection is not necessary.

### Brief description of the drawings:

These and other aspects of the invention will be apparent from and elucidated with reference to the embodiments described hereinafter.

The invention will now be described, by way of example, based on embodiments with reference to the accompanying drawings.

In the drawings:
- Fig. 1: shows a principal sketch of the wound cleaning device according to a first embodiment in cross section **(A),** and in a perspective view **(B).**
- Fig. 2: shows a principal sketch of the base station according to a first embodiment in cross section **(A)**, and in a perspective view **(B).**
- Fig. 3: shows a principal sketch of the circuit diagram of the bioimpedance measuring principle **(A),** and a possible location of the sensing electrode an the cleaning surface of the cleaning attachment **(B).**
- Fig. 4: shows a sectional view of the hand-held device **(A)** and the tip of the hand-held device together with the cleaning attachment **(B)** in a perspective view.

### Detailed description of embodiments:

Various embodiments of the invention will now be described by means of the Figures.

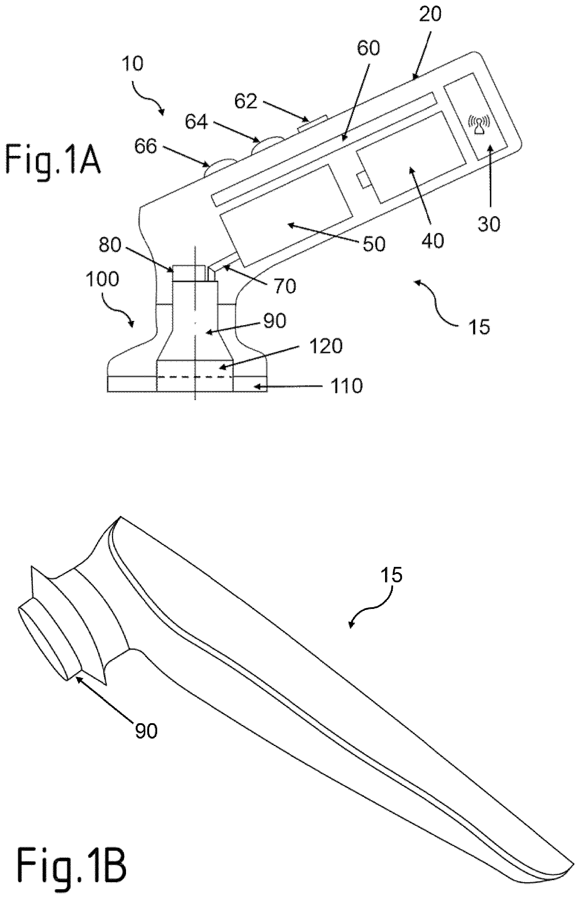

Fig. 1 shows in **(A)** a principal sketch of the wound cleaning device 10 with the hand-held device 15 comprising of a housing 20 with an integrated rechargeable battery 40 which can be charged externally by use of the loading coil 30, a control electronics 60 including a frequency generator coupled to the sonotrode 90; an electric motor 50 connected via its eccentric 70 to the cleaning attachment 100, whereby the transmitted power is measured by the power sensor 80. The housing includes a battery indicator 62, a modus switch 64 and a on/off switch 66. The cleaning attachment 100 has a central opening with liquid reservoir 120 filled with cleaning liquid 130 as ultrasound transmitting and cavitation bubbles generating medium. The cleaning attachment 100 has a cleaning surface in form of a textile pad 110. In **(B)** a prototype of the hand-held device 15 is shown (without cleaning attachment) with the cylindrical sonotrode 90 at the head of the device. The elongated housing tapers to the end so that it can be easily hold in a controlled manner.

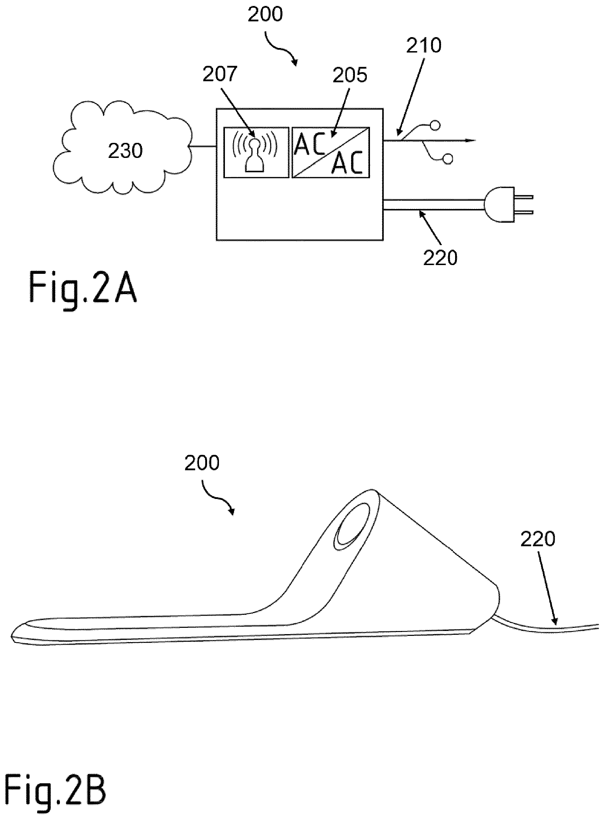

Fig. 2 shows in (A) a principal sketch of the base station 200 according to a first embodiment in cross section. Hereby, base station comprises a loading coil 207 and a power supply unit 205, and is further equipped with a power connection 220 and an USB port 210. When the hand-held device 15 (not shown here, see Fig. 1A,B) is plugged to the base station 200, the rechargeable battery 40 of the hand-held device 15 can be loaded by wireless charging 230. In the same instance, information can be exchanged wirelessly 230 between the hand-held device 15 and the base station 200. The USB port 210 of the base station can be used to store electronic data (e.g. of treatment parameters) or can be used to provide the base station with data for transmission to the hand-held device (e.g. software update). Figure 2 shows in **(B)** a perspective view of a prototypic base station 200 with power connection 220 and a recess for accepting the hand-held device (not shown).

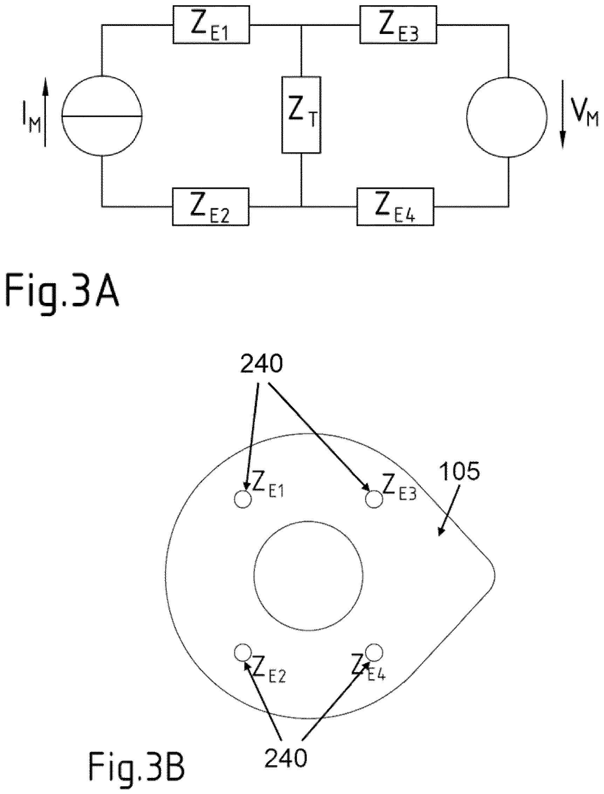

Fig. 3 shows in (A) a principal sketch of the circuit diagram of the bioimpedance measuring principle with four sensing electrodes ZE1, ZE2, ZE3, and ZE4, and in **(B)** a possible location of the sensing electrodes ZE1, ZE2, ZE3, and ZE4, an the drop-contoured cleaning surface of the cleaning attachment 105.

Fig. 4 shows a sectional view **(A)** or a perspective view **(B)** of a prototypic cleaning device, whereby the electric motor 50 and sonotrode 90 together with the needle roller bearings 75 is shown. The battery indicator 62, a modus switch 64 and a on/off switch 66 can be partially seen. As shown in **(B)** the hand-held device is connected by a snap-fit connection 106 with the replaceable cleaning attachment 105 having a central cylindrical clearance hole fitting in size and location to the underlying sonotrode 90 and further containing a drop-contoured cleaning surface textile pad 110.

While the invention has been illustrated and described in detail in the drawings and the foregoing description, such illustration and description are to be considered illustrative or exemplary and not restrictive.

From reading the present disclosure, other modifications will be apparent to persons skilled in the art. Such modifications may involve other features which are already known in the art and which may be used instead of or in addition to features already described herein.

Variations to the disclosed embodiments can be understood and effected by those skilled in the art, from a study of the drawings, the disclosure and the appended claims. In the claims, the word "comprising" does not exclude other elements or steps, and the indefinite article "a" or "an" does not exclude a plurality of elements or steps. The mere fact that certain measures are recited in mutually different dependent claims does not indicate that a combination of these measures cannot be used to advantage.

Any reference signs in the claims should not be construed as limiting the scope thereof.

### List of reference signs:

- 10: medical device
- 15: hand-held device
- 20: housing
- 30: loading coil
- 40: rechargeable battery as power source
- 50: electric motor
- 60: control electronics including frequency generator
- 62: battery indicator
- 64: modus switch
- 66: on/off switch
- 70: eccentric
- 75: needle roller bearings
- 80: power sensor
- 90: sonotrode
- 100: cleaning attachment
- 105: cleaning attachment with drop-contour cleaning surface
- 106: snap-fit connection for the cleaning attachment
- 110: textile pad as cleaning surface
- 120: liquid reservoir
- 130: cleaning liquid as ultrasound-transmitting medium
- 200: charging unit as base station
- 205: power supply unit
- 207: loading coil of the base unit
- 210: USB port
- 220: power connection
- 230: wireless communication and charging
- 240: sensing electrodes for determination of bio-impedance

## Claims

1. Medical device (10) for wound cleaning in the form of a hand-held device (15) comprising:
(a) an ultrasound generating unit comprising a frequency generator and a sonotrode (90);
(b) optionally an electric motor (50) for driving a cleaning attachment (100);
(c) a power source for operating the electric motor (50) and/or the sonotrode (90);
and a cleaning attachment (100)
- which is reversibly attachable to the hand-held device (15) and drivable by the electric motor (50) and/or the sonotrode (90);
- comprises an abrasive unit; and
- is devoid of a sonotrode;
whereby the sonotrode (90) is arranged to transfer the ultrasound energy onto the tissue to be treated.

2. Medical device (10) for wound cleaning according to claim 1, whereby the hand-held device (15) has a water-tight housing (20) in which the ultrasound-generating unit, the optional electric motor (50) and the power source are integrated.

3. Medical device (10) for wound cleaning according to claim 1 or 2, whereby the electric motor (50) by use of additional drive components is arranged to generate an oscillating, oscillatory-rotating or rotating motion of the cleaning attachment (100), and whereby the electric motor (50) is preferably configurable with regard to frequency or speed.

4. Medical device (10) for wound cleaning according to claim 1 to 3, whereby the electric motor (50) by use of additional drive components is arranged to generate a combined vertical and horizontal motion of the cleaning attachment (100).

5. Medical device (10) for wound cleaning according to any of the above claims, whereby the abrasive unit forms a cleaning surface and is designed as textile pad, foam, sponge, brush head, lamellar structure, rubber pad or a combination thereof.

6. Medical device (10) for wound cleaning according to any of the above claims, whereby the additional drive components comprise an eccentric (70), a cam lever or a gear.

7. Medical device (10) for wound cleaning according to any of the above claims, whereby the cleaning attachment (100) is reversibly attachable to the hand-held device (15) by use of a snap-fit, a gear rim, a magnetic lock or a bayonet connection, whereby a snap fit is preferred.

8. Medical device (10) for wound cleaning according to any of the above claims, whereby the surface of the sonotrode (90) defining the radiation direction is coplanar to the cleaning surface of the cleaning attachment (100).

9. Medical device (10) for wound cleaning according to any of the above claims, whereby the sonotrode (90) works according the magneto-restrictive or piezo-ceramic principle and is arranged for the generation of ultrasound frequencies in the range of between 16 kHz and 1 GHz, preferable between 20 kHz to 100 kHz.

10. Medical device (10) for wound cleaning according to any of the above claims, whereby the cleaning attachment (100) comprises an opening giving access to the sonotrode (90) and whereby the cleaning attachment (100) is preferably of annular design and/or the cleaning attachment (100) has an irregular, noncircular outer contour being more preferably a drop-like outer contour.

11. Medical device (10) for wound cleaning according to claim 10, whereby the opening is at least partially filled with a fluid-absorbing structure or covered by a fluid-absorbing structure, whereby the fluid-absorbing structure is preferably a grid, foam, sponge, or a textile fabric.

12. Medical device (10) for wound cleaning according to any of the above claims, whereby the hand-held device (15) or the cleaning attachment (100) comprises one or more of the following components:
(a) a means for identification of the cleaning attachment (100);
(b) an On/Off switch (66);
(c) a device for controlling the operation mode;
(d) a device for measuring the application duration;
(e) a sensor for measuring the contact force;
(f) a sensor for measuring the mechanical work;
(g) a sensor for measuring the bio-impedance;
(h) a temperature sensor;
(i) a sensor for measuring the released ultrasound energy;
(j) an acoustic signaler;
(k) a visual signaler;
(i) a UV-light source;
(m) a display unit for displaying operational parameters;
(n) unit for wireless communication;
(o) a liquid container with integrated rinsing;
(p) a housing (20) having rounded corners for reducing microbiological contamination;
(r) port for wired connection.

13. Medical device (10) for wound cleaning according to any of the above claims, whereby the energy source is an accumulator, preferably loadable by an inductive charging unit.

14. Medical device (10) for wound cleaning according to any of the above claims, whereby the cleaning attachment (100) comprises a hinge, which is positioned such during use the attachment can pivot freely with regard to the hand-held device (15), around a pivot axis (Ap) that extend substantially perpendicular to the oscillation and/or rotation axis (AoR) of the cleaning attachment (100).

15. Wound cleaning system consisting of a medical device for wound cleaning according to any of the above claims and a transmission medium in form of a gel or an aqueous fluid for transmitting the ultrasound waves onto the tissue to be treated.

## Patentansprüche

1. Medizinische Vorrichtung (10) für die Wundreinigung in Form eines Handgeräts (15), welche:
(a) eine Ultraschall erzeugende Einheit, die einen Frequenzgenerator und eine Sonotrode (90) umfasst,
(b) optional einen Elektromotor (50) für den Antrieb eines Reinigungsaufsatzes (100),
(c) eine Stromquelle für den Betrieb des Elektromotors (50) und/oder der Sonotrode (90) und
einen Reinigungsaufsatz (100) umfasst,
- der sich reversibel an dem Handgerät (15) befestigen und von dem Elektromotor (50) und/oder der Sonotrode (90) antreiben lässt,
- eine Abrasionseinheit umfasst und
- keine Sonotrode besitzt,
wobei die Sonotrode (90) so ausgestaltet ist, dass die Energie des Ultraschalls in das zu behandelnde Gewebe übertragen wird.

2. Medizinische Vorrichtung (10) für die Wundreinigung nach Anspruch 1, wobei das Handgerät (15) ein wasserdichtes Gehäuse (20) besitzt, in welches die Ultraschall erzeugende Einheit, der optionale Elektromotor (50) und die Stromquelle integriert sind.

3. Medizinische Vorrichtung (10) für die Wundreinigung nach Anspruch 1 oder 2, wobei der Elektromotor (50) durch Verwendung zusätzlicher Antriebskomponenten so ausgestaltet ist, dass er eine schwingende, schwingend-rotierende oder rotierende Bewegung des Reinigungsaufsatzes (100) erzeugt, und wobei der Elektromotor (50) vorzugsweise hinsichtlich Frequenz oder Geschwindigkeit einstellbar ist.

4. Medizinische Vorrichtung (10) für die Wundreinigung nach den Ansprüchen 1 bis 3, wobei der Elektromotor (50) durch Verwendung zusätzlicher Antriebskomponenten so ausgestaltet ist, dass er eine kombinierte vertikale und horizontale Bewegung des Reinigungsaufsatzes (100) erzeugt.

5. Medizinische Vorrichtung (10) für die Wundreinigung nach einem der vorhergehenden Ansprüche, wobei die Abrasionseinheit eine Reinigungsfläche bildet und als eine textile Auflage, ein Schaumstoff, ein Schwamm, ein Bürstenkopf, eine Lamellenstruktur, eine Gummiauflage oder eine Kombination davon ausgestaltet ist.

6. Medizinische Vorrichtung (10) für die Wundreinigung nach einem der vorhergehenden Ansprüche, wobei die zusätzlichen Antriebskomponenten einen Exzenter (70), einen Nockenhebel oder ein Getriebe umfassen.

7. Medizinische Vorrichtung (10) für die Wundreinigung nach einem der vorhergehenden Ansprüche, wobei der Reinigungsaufsatz (100) durch die Verwendung eines Schnappverschlusses, eines Zahnkranzes, eines Magnet- oder Bajonettverschlusses an dem Handgerät (15) reversibel zu befestigen ist, wobei der Schnappverschluss bevorzugt ist.

8. Medizinische Vorrichtung (10) für die Wundreinigung nach einem der vorhergehenden Ansprüche, wobei die Oberfläche der Sonotrode (90), welche die Beschallungsrichtung definiert, zu der Reinigungsfläche des Reinigungsaufsatzes (100) coplanar ist.

9. Medizinische Vorrichtung (10) für die Wundreinigung nach einem der vorhergehenden Ansprüche, wobei die Sonotrode (90) nach dem magnetorestriktiven oder piezokeramischen Prinzip funktioniert und für die Erzeugung von Ultraschallfrequenzen in einem Bereich von zwischen 16 kHz und 1 GHz, vorzugsweise zwischen 20 kHz und 100 kHz, ausgestaltet ist.

10. Medizinische Vorrichtung (10) für die Wundreinigung nach einem der vorhergehenden Ansprüche, wobei der Reinigungsaufsatz (100) eine Öffnung umfasst, die Zugang zu der Sonotrode (90) gewährt, und wobei der Reinigungsaufsatz (100) vorzugsweise ringförmig ausgestaltet ist und/oder der Reinigungsaufsatz (100) eine unregelmäßige, nichtzirkuläre Außenkontur besitzt, die besonders bevorzugt eine tropfenförmige Außenkontur ist.

11. Medizinische Vorrichtung (10) für die Wundreinigung nach Anspruch 10, wobei die Öffnung wenigstens teilweise mit einer flüssigkeitsabsorbierenden Struktur gefüllt oder von einer flüssigkeitsabsorbierenden Struktur bedeckt ist,
wobei die flüssigkeitsabsorbierende Struktur vorzugsweise ein Gitter, Schaumstoff, Schwamm oder Textilstoff ist.

12. Medizinische Vorrichtung (10) für die Wundreinigung nach einem der vorhergehenden Ansprüche, wobei das Handgerät (15) oder der Reinigungsaufsatz (100) eine oder mehrere der folgenden Komponenten umfasst:
(a) ein Mittel zum Erkennen des Reinigungsaufsatzes (100),
(b) einen Ein/Aus-Schalter (66),
(c) ein Mittel zum Regeln der Arbeitsweise,
(d) ein Mittel zum Messen der Verwendungsdauer,
(e) einen Sensor zum Messen der Andruckkraft,
(f) einen Sensor zum Messen der mechanischen Arbeit,
(g) einen Sensor zum Messen der Bioimpedanz,
(h) einen Temperatursensor,
(i) einen Sensor zum Messen der abgegebenen Energie des Ultraschalls,
(j) einen akustischen Signalgeber,
(k) einen visuellen Signalgeber,
(l) eine UV-Lichtquelle,
(m) ein Display zum Anzeigen von Funktionsparametern,
(n) eine Einheit für die drahtlose Kommunikation,
(o) einen Flüssigkeitsbehälter mit integrierter Spülung,
(p) ein Gehäuse (20) mit abgerundeten Ecken zur Verminderung der mikrobiologischen Kontamination und
(r) einen Anschluss für eine Elektrokabelverbindung.

13. Medizinische Vorrichtung (10) für die Wundreinigung nach einem der vorhergehenden Ansprüche, wobei die Energiequelle ein Akkumulator ist, der vorzugsweise durch eine induktive Ladeeinheit aufladbar ist.

14. Medizinische Vorrichtung (10) für die Wundreinigung nach einem der vorhergehenden Ansprüche, wobei der Reinigungsaufsatz (100) ein Gelenk umfasst, das derart angeordnet ist, dass während der Verwendung der Reinigungsaufsatz sich in Bezug auf das Handgerät (15) frei um eine Drehachse (Ap) drehen kann, die im Wesentlichen senkrecht zu der Schwingungs- und/oder Rotationsachse (AoR) des Reinigungsaufsatzes (100) verläuft.

15. Wundreinigungssystem, das aus einer medizinischen Vorrichtung für die Wundreinigung nach einem der vorhergehenden Ansprüche und einem Übertragungsmedium in Form eines Gels oder einer wässrigen Flüssigkeit zum Übertragen der Ultraschallwellen in das zu behandelnde Gewebe besteht.

## Revendications

1. Dispositif médical (10) destiné au nettoyage de plaies sous la forme d'un dispositif portatif (15) comprenant :
(a) une unité de génération d'ultrasons comprenant un générateur de fréquence et une sonotrode (90) ;
(b) éventuellement, un moteur électrique (50) pour entraîner un accessoire de nettoyage (100) ;
(c) une source d'énergie pour faire fonctionner le moteur électrique (50) et/ou la sonotrode (90) ;
et un accessoire de nettoyage (100)
- qui peut être attaché de manière réversible au dispositif portatif (15) et qui peut être entraîné par le moteur électrique (50) et/ou la sonotrode (90) ;
- comprend une unité abrasive ; et
- est dépourvu de sonotrode ;
moyennant quoi la sonotrode (90) est conçue de manière à transférer l'énergie ultrasonore sur le tissu à traiter.

2. Dispositif médical (10) destiné au nettoyage de plaies selon la revendication 1, moyennant quoi le dispositif portatif (15) a un boîtier étanche (20) dans lequel sont intégrés l'unité de génération d'ultrasons, le moteur électrique éventuel (50) et la source d'énergie.

3. Dispositif médical (10) destiné au nettoyage de plaies selon la revendication 1 ou 2, moyennant quoi le moteur électrique (50), à l'aide de composants d'entraînement supplémentaires, est conçu pour générer un mouvement oscillant, oscillant-rotatif ou rotatif de l'accessoire de nettoyage (100), et moyennant quoi le moteur électrique (50) peut de préférence être configuré en termes de fréquence ou de vitesse.

4. Dispositif médical (10) destiné au nettoyage de plaies selon les revendications 1 à 3, moyennant quoi le moteur électrique (50), à l'aide de composants d'entraînement supplémentaires, est conçu pour générer un mouvement vertical et horizontal combiné de l'accessoire de nettoyage (100).

5. Dispositif médical (10) destiné au nettoyage de plaies selon l'une quelconque des revendications précédentes, moyennant quoi l'unité abrasive forme une surface de nettoyage et est conçue sous forme de tampon textile, de mousse, d'éponge, de tête de brosse, de structure lamellaire, de tampon en caoutchouc ou d'une combinaison de ceux-ci.

6. Dispositif médical (10) destiné au nettoyage de plaies selon l'une quelconque des revendications précédentes, moyennant quoi les composants d'entraînement supplémentaires comprennent un excentrique (70), un levier à came ou un engrenage.

7. Dispositif médical (10) destiné au nettoyage de plaies selon l'une quelconque des revendications précédentes, moyennant quoi l'accessoire de nettoyage (100) peut être attaché de manière réversible au dispositif portatif (15) à l'aide d'un système d'encliquetage, d'une couronne dentée, d'une fermeture magnétique ou d'une liaison à baïonnette, moyennant quoi le système d'encliquetage est préféré.

8. Dispositif médical (10) destiné au nettoyage de plaies selon l'une quelconque des revendications précédentes, moyennant quoi la surface de la sonotrode (90) définissant la direction de rayonnement est coplanaire à la surface de nettoyage de l'accessoire de nettoyage (100).

9. Dispositif médical (10) destiné au nettoyage de plaies selon l'une quelconque des revendications précédentes, moyennant quoi la sonotrode (90) fonctionne selon le principe magnéto-restrictif ou piézo-céramique et est conçue pour générer des fréquences ultrasonores comprises entre 16 kHz et 1 GHz, de préférence, entre 20 kHz et 100 kHz.

10. Dispositif médical (10) destiné au nettoyage de plaies selon l'une quelconque des revendications précédentes, moyennant quoi l'accessoire de nettoyage (100) comprend une ouverture donnant accès à la sonotrode (90) et moyennant quoi l'accessoire de nettoyage (100) est de préférence de conception annulaire et/ou l'accessoire de nettoyage (100) a un contour extérieur irrégulier, non circulaire, étant plus préférablement un contour extérieur en forme de goutte.

11. Dispositif médical (10) destiné au nettoyage de plaies selon la revendication 10, moyennant quoi l'ouverture est au moins partiellement remplie d'une structure absorbant les fluides ou recouverte d'une structure absorbant les fluides, moyennant quoi la structure absorbant les fluides est de préférence une grille, une mousse, une éponge ou un tissu textile.

12. Dispositif médical (10) destiné au nettoyage de plaies selon l'une quelconque des revendications précédentes, moyennant quoi le dispositif portatif (15) ou l'accessoire de nettoyage (100) comprend un ou plusieurs des composants suivants :
(a) un moyen d'identification de l'accessoire de nettoyage (100) ;
(b) un interrupteur marche/arrêt (66) ;
(c) un dispositif de commande du mode de fonctionnement ;
(d) un dispositif de mesure de la durée d'application ;
(e) un capteur pour mesurer la force de contact ;
(f) un capteur pour mesurer le travail mécanique ;
(g) un capteur pour mesurer la bio-impédance ;
(h) un capteur de température ;
(i) un capteur pour mesurer l'énergie ultrasonore libérée ;
(j) un signaleur acoustique ;
(k) un signaleur visuel ;
(l) une source de lumière UV ;
(m) une unité d'affichage pour afficher des paramètres opérationnels ;
(n) une unité de communication sans fil ;
(o) un réservoir de liquide avec rinçage intégré ;
(p) un boîtier (20) ayant des angles arrondis pour réduire la contamination microbiologique ;
(r) un port pour une connexion câblée.

13. Dispositif médical (10) destiné au nettoyage de plaies selon l'une quelconque des revendications précédentes, moyennant quoi la source d'énergie est un accumulateur, de préférence pouvant être chargé par une unité de charge inductive.

14. Dispositif médical (10) destiné au nettoyage de plaies selon l'une quelconque des revendications précédentes, moyennant quoi l'accessoire de nettoyage (100) comprend une charnière, qui est positionnée de manière que, pendant l'utilisation, l'accessoire peut pivoter librement par rapport au dispositif portatif (15), autour d'un axe de pivotement (Ap) qui s'étend sensiblement perpendiculairement à l'axe d'oscillation et/ou de rotation (AoR) de l'accessoire de nettoyage (100).

15. Système de nettoyage de plaies constitué d'un dispositif médical destiné au nettoyage de plaies selon l'une quelconque des revendications précédentes et d'un milieu de transmission sous forme de gel ou de fluide aqueux pour transmettre les ondes ultrasonores sur le tissu à traiter.
